# EUROPEAN PATENT APPLICATION

(11) **EP 3 193 173 A1**
(43) Date of publication of application: **19.07.2017**
(21) Application number: 16151350.2
(22) Date of filing: 14.01.2016
(51) Int. Cl.: G01N 33/574, G01N 33/564

(54) **SEROLOGICAL AUTOANTIBODIES AS BIOMARKER FOR COLORECTAL CANCER**

(71) Applicant: Deutsches Krebsforschungszentrum, Stiftung des öffentlichen Rechts, 69120 Heidelberg (DE)
(72) Inventor: Brenner, Hermann, 69257 Wiesenbach (DE); Chen, Hongda, 68161 Mannheim (DE); Pawlita, Michael, 74927 Eschelbronn (DE); Jäger, Dirk, 69121 Heidelberg (DE); Zörnig, Inka, 60528 Frankfurt (DE); Eichmüller, Stefan, 68239 Mannheim (DE); Waterboer, Tim, 69126 Heidelberg (DE); Butt, Julia, 69155 Heidelberg (DE)
(74) Representative: Krauss, Jan

(57) **Abstract**

The present invention pertains to a new method for the diagnosis, prognosis, stratification and/or monitoring of a therapy, of colorectal cancer in a patient. The method is based on the determination of the level or concentration of one or more autoantibodies specific for a tumor associated antigen selected from TP53, IMPDH2, MAGEA4, RPL13, MDM2, CTAG1, RPH3AL, ANXA4, MTDH, TPM3_isol, ERBB2_C_term, IGF2BP1, MIA, DDX53, HMGN3, CALU, KLK3_isol, REG3A, CTAG2, ERBB2_N_term, and MAGEA3. The new serological antibody marker panel of the invention allows diagnosing and even stratifying colorectal cancer diseases. Furthermore provided are diagnostic kits for performing the non-invasive methods of the invention.

## Description

### FIELD OF THE INVENTION

The present invention pertains to a new method for the diagnosis, prognosis, stratification and/or monitoring of a therapy, of colorectal cancer in a patient. The method is based on the determination of the level or concentration of one or more autoantibodies specific for a tumor associated antigen selected from TP53, IMPDH2, MAGEA4, RPL13, MDM2, CTAG1, RPH3AL, ANXA4, MTDH, TPM3_iso1, ERBB2_C_term, IGF2BP1, MIA, DDX53, HMGN3, CALU, KLK3_iso1, REG3A, CTAG2, ERBB2_N_term, and MAGEA3. The new serological antibody marker panel of the invention allows diagnosing and even stratifying colorectal cancer diseases. Furthermore provided are diagnostic kits for performing the non-invasive methods of the invention.

### DESCRIPTION

With approximately 1.4 million new cases and 700,000 deaths occurring in 2012, colorectal cancer (CRC) is the third most cancer and the fourth most common cause of death from cancer worldwide. Stage at diagnosis is the most important prognostic factor, with 5-year relative survival ranging from >90% for patients with localized CRC to approximately 10% for patients with distant tumor spread. Randomized trials and observational studies have shown a large potential for reduction of CRC incidence and mortality by endoscopic or stool-based screening tests, such as sigmoidoscopy, colonoscopy, guaiac-based or immunochemical fecal occult blood tests.

An alternative approach for cancer screening might be blood-based screening tests. Due to their minimally invasive nature and straightforward implementation in routine medical examinations, blood tests might achieve high levels of adherence when applied in population-based screening.

The mechanism behind the humoral immune responses toward TAAs is complex and not fully understood. Production of autoantibodies could be induced in responses to over-expression, mutations, or abnormal posttranslational modifications of proteins in cancer cells. Although the sensitivity of single autoantibodies for cancer detection seems to be low, higher sensitivities might be achieved by joint testing for multiple autoantibodies.

Clearly there remains a significant unmet need for more and better compositions and methods for diagnosing cancer and for cancer screening, including additional biomarkers and conveniently practiced approaches that are capable of detecting cancer in a subject at an earlier point in the progression of the disease than is currently possible (e.g., in a pre-diagnostic subject), and preferably further including conveniently practiced methods that permit monitoring the severity of disease, progression of disease and/or disease responsiveness to a therapeutic course. Effective early diagnosis of cancer would reduce the tumor burden that is typically present at the inception of surgical, chemotherapeutic, immunotherapeutic, molecular therapeutic and/or radiation-based therapies, relative to the tumor burden presently confronting the clinician when current conventional diagnostics are relied upon, and could have a significant impact on cancer related mortality.

The above problem is solved in a first aspect by a non-invasive method for, or for aiding, a diagnosis, prognosis, stratification and/or monitoring of a therapy, of a cancer disease in a subject, comprising the steps of:
(a) Providing a biological sample from the subject,
(b) Determining in the sample the level of at least one autoantibody selected from anti-MAGEA4, anti-TP53, anti-IMPDH2, anti-RPL13, anti-MDM2, anti-CTAG1, anti-RPH3AL, anti-ANXA4, anti-MTDH, anti-TPM3_iso1, anti-ERBB2_C_term, anti-IGF2BP1, anti-MIA, anti-DDX53, anti-HMGN3, anti-CALU, anti-KLK3_iso1, anti-REG3A, anti-CTAG2, anti-ERBB2_N_term, or anti-MAGEA3,
wherein a differential level of the at least one autoantibody in the biological sample from the subject as determined in step (b) compared to a healthy control sample or reference value is indicative for the presence of the cancer disease in the subject.

The level of one or more autoantibodies as mentioned is preferably detected using the binding of the autoantibody to the respective tumor associated antigen (TAA) protein.

Thus, the invention in one embodiment, provides a non-invasive method for diagnosing cancer in a subject, comprising (a) contacting (i) one or more autoantibodies from a biological sample from the subject, and (ii) at least one isolated cancer indicator protein (a TAA), under conditions and for a time sufficient for detecting specific binding of at least one autoantibody from the biological sample to one or more of said cancer indicator proteins, and therefrom identifying presence of the cancer in the subject.

In another embodiment, there is provided a non-invasive screening method for cancer, comprising (a) contacting (i) one or more autoantibodies from a biological fluid from each subject of one or a plurality of subjects, and (ii) at least one isolated cancer indicator proteins (TAA), under conditions and for a time sufficient for detecting specific binding of at least one autoantibody from the biological sample to one or more of said cancer indicator proteins, wherein detection of specific binding indicates the subject has cancer, and thereby screening for cancer.

In another embodiment there is provided a non-invasive method for diagnosing cancer in a subject, comprising (a) contacting (i) one or more autoantibodies from a biological sample from the subject, and (ii) an isolated protein or polypeptide that comprises one or more antigenic epitopes of one or more cancer indicator proteins, under conditions and for a time sufficient for detecting specific binding of at least one autoantibody from the biological sample to one or more of said antigenic epitopes, and therefrom identifying presence of cancer in the subject.

In another embodiment there is provided a non-invasive screening method for cancer, comprising (a) contacting (i) one or more antibodies from a biological sample from each subject of one or a plurality of subjects, and (ii) an isolated protein or polypeptide that comprises one or more antigenic epitopes of one or more cancer indicator proteins, under conditions and for a time sufficient for detecting specific binding of at least one autoantibody from the biological sample to one or more of said antigenic epitopes, wherein detection of specific binding indicates the subject has cancer, and thereby screening for cancer.

The cancer indicator protein is a TAA protein, and preferably selected from MAGEA4, TP53, IMPDH2, RPL13, MDM2, CTAG1, RPH3AL, ANXA4, MTDH, TPM3_iso_1, ERBB2_C_term, IGF2BP1, MIA, DDX53, HMGN3, CALU, KLK3_iso1, REG3A, CTAG2, ERBB2_N_term, or MAGEA3. The autoantibody is correspondingly selected from anti-MAGEA4, anti-TP53, anti-IMPDH2, anti-RPL13, anti-MDM2, anti-CTAG1, anti-RPH3AL, anti-ANXA4, anti-MTDH, anti-TPM3_iso1, anti-ERBB2_C_term, anti-IGF2BP1, anti-MIA, anti-DDX53, anti-HMGN3, anti-CALU, anti-KLK3_iso1, anti-REG3A, anti-CTAG2, anti-ERBB2_N_term, or anti-MAGEA3.

The methods of the invention preferably comprise determining in the sample the level of autoantibodies anti-TP53 and anti-IMPDH2.

More preferably the methods of the invention comprise determining in the sample the level of autoantibodies anti-TP53, anti-IMPDH2 and anti-MDM2.

Even more preferably the methods of the invention may comprise determining in the sample the level of the autoantibodies anti-MAGEA4, anti-TP53, anti-IMPDH2, and anti-MDM2.

Also preferred are the methods of the invention wherein the methods comprise determining in the sample the level of the autoantibodies anti-MAGEA4, anti-TP53, anti-IMPDH2, anti-MDM2, and anti-CTAG1.

Also preferred are the methods of the invention wherein the methods comprise determining in the sample the level of the autoantibodies anti-MAGEA4, anti-TP53, anti-IMPDH2, and anti-MDM2, anti-CTAG1 and anti-MTDH.

Further embodiments of the invention pertain to methods wherein the level of anti-MAGEA4 autoantibodies are determined in the sample, together with at least one further autoantibody selected from anti-TP53, anti-IMPDH2, anti-RPL13, anti-MDM2, anti-CTAG1, anti-RPH3AL, anti-ANXA4, anti-MTDH, anti-TPM3_iso1, anti-ERBB2_C_term, anti-IGF2BP1, anti-MIA, anti-DDX53, anti-HMGN3, anti-CALU, anti-KLK3_iso1, anti-REG3A, anti-CTAG2, anti-ERBB2_N_term, or anti-MAGEA3.

In preferred embodiments level shall be understood to refer to a concentration. Other embodiments relate to methods wherein a differential level of an autoantibody is a higher level compared to the control or reference level.

A "diagnosis" or the term "diagnostic" in context of the present invention means identifying the presence or nature of a pathologic condition. Diagnostic methods differ in their sensitivity and specificity. The "sensitivity" of a diagnostic assay is the percentage of diseased individuals who test positive (percent of "true positives"). Diseased individuals not detected by the assay are "false negatives." Subjects who are not diseased and who test negative in the assay, are termed "true negatives." The "specificity" of a diagnostic assay is 1 minus the false positive rate, where the "false positive" rate is defined as the proportion of those without the disease who test positive. While a particular diagnostic method may not provide a definitive diagnosis of a condition, it suffices if the method provides a positive indication that aids in diagnosis.

The term "prognosis" refers to a forecast as to the probable outcome of the disease as well as the prospect of recovery from the disease as indicated by the nature and symptoms of the case. Accordingly, a negative or poor prognosis is defined by a lower post-treatment survival term or survival rate. Conversely, a positive or good prognosis is defined by an elevated post-treatment survival term or survival rate. Usually prognosis is provided as the time of progression free survival or overall survival.

The term "stratification" for the purposes of this invention refers to the advantage that the method according to the invention renders possible decisions for the treatment and therapy of the patient, whether it is the hospitalization of the patient, the use, effect and/or dosage of one or more drugs, a therapeutic measure or the monitoring of a course of the disease and the course of therapy or etiology or classification of a disease, e.g., into a new or existing subtype or the differentiation of diseases and the patients thereof. Particularly with regard to colorectal cancer, "stratification" means in this context a classification of a colorectal cancer as early or late stage colorectal cancer.

The term "monitoring a therapy" means for the purpose of the present invention to observe disease progression in a subject who receives a cancer therapy. In other words, the subject during the therapy is regularly monitored for the effect of the applied therapy, which allows the medical practitioner to estimate at an early stage during the therapy whether the prescribed treatment is effective or not, and therefore to adjust the treatment regime accordingly.

As used herein, the term "subject" or "patient" refers to any animal (e.g., a mammal), including, but not limited to, humans, non-human primates, rodents, and the like, which is to be the recipient of a particular treatment. Typically, the terms "subject" and "patient" are used interchangeably herein in reference to a human subject. As used herein, the term "subject suspected of having cancer" refers to a subject that presents one or more symptoms indicative of a cancer (e.g., a noticeable lump or mass). A subject suspected of having cancer may also have one or more risk factors. A subject suspected of having cancer has generally not been tested for cancer. However, a "subject suspected of having cancer" encompasses an individual who has received an initial diagnosis (e.g., a CT scan showing a mass) but for whom the sub-type or stage of cancer is not known. The term further includes people who once had cancer (e.g., an individual in remission), and people who have cancer and are suspected to have a metastatic spread of the primary tumor. In this regard the present invention is also applicable as follow-up care for monitoring a subject for a reoccurrence of the cancer. In some preferred embodiment a pre-diagnostic subject is preferred. Pre-diagnostic is a subject if no previous positive diagnosis was conducted, or wherein after successful treatment of the disease, the subject is undergoing regular post-illness monitoring.

The term "cancer" and "cancer cells" refers to any cells that exhibit uncontrolled growth in a tissue or organ of a multicellular organism. Particular preferred cancers in context of the present invention are selected from colorectal cancer, pancreatic cancer, gastric cancer, breast cancer, lung cancer, prostate cancer, hepatocellular cancer, cervical cancer, ovarian cancer, liver cancer, bladder cancer, cancer of the urinary tract, thyroid cancer, renal cancer, carcinoma, melanoma, leukemia or brain cancer.

As used herein, the term "colorectal cancer" includes the well-accepted medical definition that defines colorectal cancer as a medical condition characterized by cancer of cells of the intestinal tract below the small intestine (i.e., the large intestine (colon), including the cecum, ascending colon, transverse colon, descending colon, sigmoid colon, and rectum). Additionally, as used herein, the term "colorectal cancer" also further includes medical conditions, which are characterized by cancer of cells of the duodenum and small intestine (jejunum and ileum).

A preferred cancer in context of the present invention is a colorectal cancer in an early stage, such as UICC (Union for International Cancer Control) TNM (tumor-node-metastasis) stage 0/I or II. In other embodiments the colorectal cancer is a late stage CRC, such as UICC TNM stage III or IV.

As used herein, the terms "gastric cancer" or "stomach cancer" refer to cancers of the stomach. The most common types of gastric cancer are carcinomas, such as but not limited to, adenocarcinomas, affecting the epithelial cells of the stomach. Stomach cancers may additionally include, for example, sarcomas affecting the connective tissue of the stomach and blastomas affecting the blast tissue of the stomach.

The term "pancreatic cancer" encompasses benign or malignant forms of pancreatic cancer, as well as any particular type of cancer arising from cells of the pancreas (e.g., duct cell carcinoma, acinar cell carcinoma, papillary carcinoma, adenosquamous carcinoma, undifferentiated carcinoma, mucinous carcinoma, giant cell carcinoma, mixed type pancreatic cancer, small cell carcinoma, cystadenocarcinoma, unclassified pancreatic cancers, pancreatoblastoma, and papillary-cystic neoplasm, and the like.).

The term "biological sample" as used herein refers to a sample that was obtained and may be assayed for any one of the autoantibodies as disclosed with the present invention, or their gene expression. The biological sample can include a biological fluid (e.g., blood, cerebrospinal fluid, urine, plasma, serum), tissue biopsy, and the like. In some embodiments, the sample is a tissue sample, for example, tumor tissue, and may be fresh, frozen, or archival paraffin embedded tissue. Preferred samples for the purposes of the present invention are bodily fluids, in particular blood or serum samples.

Certain biological fluids derive from particular tissues, organs or localized regions and certain other biological fluids may be more globally or systemically situated in a subject or biological source. Examples of biological fluids include blood, serum and serosal fluids, plasma, lymph, urine, cerebrospinal fluid, saliva, mucosal secretions of the secretory tissues and organs, vaginal secretions, ascites fluids such as those associated with non- solid tumors, fluids of the pleural, pericardial, peritoneal, abdominal and other body cavities, and the like. Biological fluids may also include liquid solutions contacted with a subject or biological source, for example, cell and organ culture medium including cell or organ conditioned medium, lavage fluids and the like. In certain highly preferred embodiments the biological sample is serum, and in certain other highly preferred embodiments the biological sample is plasma. In other preferred embodiments the biological sample is a cell-free liquid solution. It is contemplated that in certain embodiments the antibodies are present in the biological fluid at the time of contacting with the pre-diagnostic indicator protein(s), but the invention is not so limited and also contemplates embodiments in which the antibodies are isolated from the biological fluid prior to the step of contacting. The term "isolated" means that the material is removed from its original environment (e.g., the natural environment if it is naturally occurring). For example, a naturally occurring antibody [e.g., autoantibody), polypeptide or polynucleotide present in a living subject (e.g., a pre-diagnostic subject or a patient) is not isolated, but the same antibody, polypeptide or polynucleotide, separated from some or all of the coexisting materials in the natural system, is isolated. Such antibodies, polypeptides or polynucleotides could be part of a composition, and still be isolated in that such composition is not part of its natural environment. Isolation of antibodies may be achieved according to any of a wide variety of methodologies with which persons skilled in the art will be familiar, including biochemical and/or immunological methods.

The term "antibodies" includes immunoglobulins such as polyclonal antibodies, monoclonal antibodies, fragments thereof such as F(ab')2, and Fab fragments, as well as any naturally occurring immunoglobulin variable (V) region complementarity determining region (CDR)-containing binding partners (also including in certain embodiments antigen-binding CDR-containing T cell receptor polypeptides which are encoded by members of the immunoglobulin gene superfamily, whilst certain other embodiments expressly exclude such T cell-derived polypeptides), which are molecules that specifically bind a pre-diagnostic cancer indicator protein. As described in greater detail below, particularly preferred embodiments relate to detection in a biological fluid from a pre-diagnostic subject of antibodies that are autoantibodies, i.e., antibodies that specifically recognize and bind to "self antigenic epitopes that may also be found in the subject. Briefly, it is well accepted in the art that the immune system (e.g., adaptive immune system) is typically characterized as distinguishing foreign agents (or "non-self) agents from familiar or "self components, such that foreign agents elicit immune responses while "self components are ignored or tolerated. Exceptions to this paradigm arise, however, in the case of autoantibody generation, whereby a host immune system produces antibodies that react with "self antigens. See, e.g., Theofilopoulos and Bona, The Molecular Pathology of Autoimmune Diseases, CRC Press, Boca Raton, FL, 2002; Doria et al., Handbook of Systemic Autoimmune Diseases (VoIs. 1-9), Elsevier, NY, 2004-2008; Roitt et al., Immunology (6th Ed.), Mosby, NY, 2001 , Ch. 26. Accordingly and as described herein, there are provided the present embodiments in which an autoantibody is detected in the cancer diagnostic methods, wherein the autoantibody detectably and specifically binds to a pre- diagnostic cancer indicator protein, which protein may be a "self" component in the pre-diagnostic subject.

Antibodies are defined to be "specific" or to be capable of specifically binding if they bind a pre-diagnostic (colorectal) cancer indicator protein (TAA) (or a polypeptide that comprises one or more antigenic epitopes of such a protein) with a Kₐ of greater than or equal to about 10⁴ M⁻¹, preferably of greater than or equal to about 10⁵ M⁻¹, more preferably of greater than or equal to about 10⁶ M⁻¹ and still more preferably of greater than or equal to about 10⁷ M⁻¹.

Affinities of binding partners or antibodies can be readily determined using conventional techniques, for example those described by Scatchard et al., Ann. N.Y. Acad. Sci. 57:660 (1949), or by surface plasmon resonance (SPR) spectroscopy, or by any of a number of other known methods for identifying and characterizing antibodies or antibody-derived proteins that specifically interact with cognate antigens via recognition and binding of antigenic epitopes. For instance, to detect an antibody that specifically binds to a pre-diagnostic cancer indicator protein (or a polypeptide that comprises one or more antigenic epitopes of such a protein), there are a variety of assay formats, including but not limited to enzyme linked immunosorbent assay (ELISA), radioimmunoassay (RIA), immunofluorimetry, immunoprecipitation, equilibrium dialysis, immunodiffusion and other techniques. See, e.g., Harlow and Lane, Antibodies: A Laboratory Manual, Cold Spring Harbor Laboratory, 1988; Weir, D. M., Handbook of Experimental Immunology, 1986, Blackwell Scientific, Boston. See, e.g., Coligan et al. (Eds.), Current Protocols in Immunology (2007 John Wiley & Sons, NY); Harlow and Lane, Antibodies: A Laboratory Manual (1988 Cold Spring Harbor Press, Cold Spring Harbor, NY); Harlow and Lane, Using Antibodies (1999 Cold Spring Harbor Press, Cold Spring Harbor, NY). Therein can also be found parameters for designing immunoassay conditions, including conditions and incubation times that will be sufficient for detection of specific binding of an antibody to its cognate antigen. Most preferred is the use of a fluorescent based multiplex immunosorbent assay as described before (Zornig I, Halama N, Lorenzo Bermejo J, Ziegelmeier C, Dickes E, Migdoll A, et al. Prognostic significance of spontaneous antibody responses against tumor-associated antigens in malignant melanoma patients. Int J Cancer. 2015;136:138-51; and Waterboer T, Sehr P, Michael KM, Franceschi S, Nieland JD, Joos TO, et al. Multiplex human papillomavirus serology based on in situ-purified glutathione s-transferase fusion proteins. Clin Chem. 2005;51:1845-53.).

As described herein, the present invention relates to detecting the presence in a biological sample of an autoantibody that specifically binds to one or more tumor associated antigenic (TAA) proteins. As used in the present invention the autoantibodies are defined according to their specificity against such TAAs. The nomenclature used here describes an autoantibody with the prefix "anti-" followed by the protein name of the TAA (for example anti-MAGE A4 describes an autoantibody specific for the protein MAGE A4). A TAA thus may include any one or more of a number of target antigens for which the presence in a biological sample from a subject of a specifically reactive cognate antibody indicates that the subject has cancer. As is well known in the immunological art, specifically-binding antibodies define antigenic epitopes on their cognate antigens, i.e., the molecular structure with which the antibody's antigen-combining site interacts.

For example, an antigenic epitope of a TAA may be defined by a region of protein primary structure (e.g., amino acid sequence), by protein secondary structure (e.g., a localized structure that is spatially defined such as may be formed by a common motif or repetitive domain, e.g, alpha-helix, beta-sheet, etc.), by protein tertiary structure (e.g., by three-dimensional structure that is created by protein folding or conformation) or by quartemary structure (e.g., by the interaction of two or more polypeptide subunits, as in a complex or oligomer). Antigenic epitopes may additionally or alternatively be defined in whole or in part by post- translational modifications to pre-diagnostic cancer indicator proteins, including by virtue of direct involvement of the post-translational modification in formation of the epitope structure and also including, e.g., conformational epitopes the presence of which may depend on the presence of a particular post-translational modification. Accordingly, certain embodiments described herein contemplate use of an intact TAA, which may bear one, two, three, four, five, six or more antigenic epitopes that can be defined by antibody reactivities, while certain other embodiments contemplate use of an isolated protein or polypeptide that comprises one or more antigenic epitopes of one or more TAA. A TAA thus may be (i) a naturally occurring protein or polypeptide, (ii) a synthetic protein or polypeptide, (iii) a recombinant protein or polypeptide, or (iv) a fusion protein or polypeptide that comprises a fusion polypeptide domain fused to the pre-diagnostic indicator protein, or to the polypeptide that comprises one or more antigenic epitopes of the TAA.

Fragments or portions of the TAA proteins or polypeptides derived therefrom may be employed in certain herein disclosed embodiments, which fragments or portions retain at least one antigenic epitope that is capable of being specifically recognized by an autoantibody from a biological sample of subject having cancer, and which may have at least 50, 60, 70, 80, 85, 90, 91, 92, 93, 94, 95, 96, 97, 98, or 99 percent identity to the TAA protein sequences. The methods to be practiced according to certain herein disclosed embodiments may involve any convenient format for interrogating a biological sample, for example a serum sample, as provided herein, for the presence of one or more autoantibodies that are capable of specifically binding to a TAA protein as provided herein. Alternatively, certain embodiments may involve any convenient format for interrogating an antibody-containing fraction that has been isolated from a biological sample, as provided herein, for the presence of one or more autoantibodies that are capable of specifically binding to a TAA protein as provided herein.

As also noted above, any of a number of convenient formats may be employed for contacting one or more autoantibodies from a sample with an TAA protein to detect specific autoantibody binding to the indicator protein, including but not limited to enzyme linked immunosorbent assay (ELISA), surface plasmon resonance (SPR) spectroscopy, western blot immunoassay, radioimmunoassay (RIA), immunofluorimetry, immunoprecipitation, equilibrium dialysis, immunodiffusion and other techniques. See, e.g., Harlow and Lane, Antibodies: A Laboratory Manual, Cold Spring Harbor Laboratory, 1988; Weir, D. M., Handbook of Experimental Immunology, 1986, Blackwell Scientific, Boston. See, e.g., Coligan et al. (Eds.), Current Protocols in Immunology (2007 John Wiley & Sons, NY); Harlow and Lane, Antibodies: A Laboratory Manual (1988 Cold Spring Harbor Press, Cold Spring Harbor, NY); Harlow and Lane, Using Antibodies (1999 Cold Spring Harbor Press, Cold Spring Harbor, NY).

The term "determining the level of" an autoantibody in a sample, control or reference, as described herein shall refer to the quantification of the presence of said autoantibody in the testes sample. For example the concentration of the autoantibody in said samples may be directly quantified via measuring the amount of autoantibody as present in the tested sample. The present invention shall not be restricted to any particular method for determining the level of a given autoantibody, but shall encompass all means that allow for a quantification, or estimation, of the level of said autoantibody, either directly or indirectly. "Level" in the context of the present invention is therefore a parameter describing the absolute amount of an autoantibody in a given sample, for example as absolute weight, volume, or molar amounts; or alternatively "level" pertains to the relative amounts, for example and preferably the concentration of said autoantibody in the tested sample, for example mol/l, g/l, g/mol etc. In preferred embodiments the "level" refers to the concentration of the tested autoantibody in g/l.

"Increase" of the level of an autoantibody in a sample compared to a control shall in preferred embodiments refer to statistically significant increase in preferred aspects of the invention.

In a preferred embodiment the method of the herein disclosed invention is performed *in vitro* or *ex vivo*. Since the herein described diagnostic methods are non-invasive the term "providing a biological" sample shall preferably not be interpreted to include a surgical procedure conducted at the subject.

One specifically preferred panel for use in context of the herein disclosed invention comprises the selected of at least 4, 5, 6, 7 or 8 autoantibody biomarkers selected from the group of anti-MAGEA4, anti-TP53, anti-IMPDH2, anti-RPL13, anti-MDM2, anti-CTAG1, anti-RPH3AL, anti-ANXA4, anti-MTDH, anti-TPM3_iso1, anti-ERBB2_C_term, anti-IGF2BP1, anti-MIA, anti-DDX53, anti-HMGN3, anti-CALU, anti-KLK3_iso1, anti-REG3A, anti-CTAG2, anti-ERBB2_N_term, or anti-MAGEA3, in the biological sample. Most preferred in this aspect is that at least the autoantibodies anti-MAGEA4 or anti-TP53, optionally any of the remaining biomarkers is included in this panel.

To date, no single blood biomarker qualifying for mass screening has been identified. The use of autoantibody markers, also in combination of multiple autoantibodies, might be a more promising approach to achieve the necessary sensitivity and specificity for application in mass screening.

The autoantibody biomarker panel as disclosed herein is particular useful in a cancer screening setting. Cancer screening in the herein disclosed invention shall refer to a procedure where a subject is for which not diagnosis was established is tested for the presence of the cancer disease. This shall not be interpreted to exclude the use of the biomarker of the invention for a diagnostic of a subject that was already diagnosed to suffer from a cancer disease. Non limiting examples for such an application are confirmation of a diagnosis, monitoring or treatment success or monitoring reoccurrence of a cancer in a subject that already received a treatment and wherein cancer is in remission or was cured.

The skilled artisan will understand that numerous methods may be used to select a threshold or reference value for a particular autoantibody or a plurality of autoantibodies. In diagnostic aspects, a threshold value may be obtained by performing the assay method on samples obtained from a population of patients having a certain type of cancer, and from a second population of subjects that do not have cancer. For prognostic or treatment monitoring applications, a population of patients, all of which have, for example, ovarian cancer, may be followed for the time period of interest (e.g., six months following diagnosis or treatment, respectively), and then dividing the population into two groups: a first group of subjects that progress to an endpoint (e.g., recurrence of disease, death); and a second group of subjects that did not progress to the end point. These are used to establish "low risk" and "high risk" population values for the marker(s) measured, respectively. Other suitable endpoints include, but are not limited to, 5-year mortality rates or progression to metastatic disease.

Once these groups are established, one or more thresholds may be selected that provide an acceptable ability to predict diagnosis, prognostic risk, treatment success, etc. In practice, Receiver Operating Characteristic curves, or "ROC" curves, are typically calculated by plotting the value of a variable versus its relative frequency in two populations (called arbitrarily "disease" and "normal" or "low risk" and "high risk" for example). For any particular marker, a distribution of marker levels for subjects with and without a disease may overlap. Under such conditions, a test does not absolutely distinguish "disease" and "normal" with 100% accuracy, and the area of overlap indicates where the test cannot distinguish "disease" and "normal." A threshold is selected, above which (or below which, depending on how a marker changes with the disease) the test is considered to be "positive" and below which the test is considered to be "negative." The area under the ROC curve is a measure of the probability that the perceived measurement may allow correct identification of a condition.

Additionally, thresholds may be established by obtaining an earlier marker result from the same patient, to which later results may be compared. In some aspects, the individuals act as their own "control group." In autoantibodies that increase with disease severity or prognostic risk, an increase over time in the same patient can indicate a worsening of disease or a failure of a treatment regimen, while a decrease over time can indicate remission of disease or success of a treatment regimen.

All numeric values are herein assumed to be modified by the term "about," whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure. In particularly preferred embodiments of the invention the term "about" may refer to a deviation of the respective numeric value of a maximum of 20% of the numerical value, however more preferred is 15%, 10%, 5% and most preferred is 4%, 3%, 2%, and most preferred is 1%.

In a preferred embodiment said sample is selected from the group consisting of body fluids or tissue, preferably wherein said body fluid sample is a blood sample, more preferably a plasma or serum sample.

Another embodiment of the invention pertains to the application of the aforementioned diagnostic marker panel of the invention and the additional performance of at least one additional cancer diagnostic assay. Preferably, in the event colorectal cancer is to be diagnosed by the methods of the present invention, a preferred embodiment requires the additional performance of at least one additional colorectal cancer diagnostic assay. The at least one additional colorectal cancer diagnostic assay preferably comprises the analysis of blood in a stool sample of the patient. Various methods are known to the skilled artisan for the detection of blood in stool. One preferred assay is the immunological detection of haemoglobin in a stool sample. Such assays are known as immunochemical fecal occult blood test (iFOBT), or fecal immunochemical test (FIT). This assay is a widely used test that detects small traces of blood in stool, derived from lesions such as tumors that bleed in the colon. The FIT is a non-invasive method that makes use of an antibody directed against hemoglobin. However, not all colon tumors bleed and therefore the FIT has a sensitivity that leaves room for improvement (Duffy M J, et al. Int J Cancer 2011; 128:3-11; van Veen W, Mali W P. [Colorectal cancer screening: advice from the Health Council of the Netherlands]. Ned Tijdschr Geneeskd 2009; 153:A1441).

In context of the present invention it was surprisingly found that a combination of a marker panel of the present invention, most preferred are the four markers anti-TP53, anti-IMPDH2, anti-MDM2 and anti-MAGEA4, with a FIT assay greatly improves sensitivity of the colorectal cancer and its precursors (i.e., advanced adenoma and non-advanced adenoma) diagnosis. Therefore, in a most preferred embodiment of the invention, the diagnostic method comprises the determining in the sample the level of the autoantibodies anti-TP53, anti-IMPDH2, anti-MDM2 and anti-MAGEA4 in a sample of the patient to be diagnosed, in combination with the use of a FIT assay.

In all aspects and embodiments of the present invention in may be preferred that the level of said at least one biomarker in said sample is determined by means of an immunological detection method based on the specific binding of an autoantibody to its respective TAA protein. Generally all means shall be comprised by the present invention which allow for a quantification of the presence of any one of the herein disclosed autoantibodies in the sample.

In yet another aspect, the invention provides kits for aiding a diagnosis of cancer, wherein the kits can be used to detect the autoantibodies of the present invention. For example, the kits can be used to detect any one or combination of autoantibodies described above, which autoantibodies are differentially present in samples of a patient having the cancer and healthy patients. The kits of the invention have many applications. For example, the kits can be used to differentiate if a subject has the cancer, or has a negative diagnosis, thus aiding a cancer diagnosis. In another example, the kits can be used to identify compounds that modulate presence of an autoantibody in *in vitro* cancer cells or *in vivo* animal models for cancer.

In another embodiment, a kit comprises (a) an antigenic protein, or antigenic fragment thereof, that specifically binds with an autoantibody of the invention; and (b) a detection reagent. Such kits can be prepared from the materials, and the previous discussion regarding the materials (e.g., antibodies, detection reagents, immobilized supports, etc.) is fully applicable to this section and need not be repeated.

The diagnostic kit of the invention in some embodiments shall comprise a panel of at least one, preferably two, three, four, five, six or seven, TAA proteins, or their antigenic fragments, selected from MAGEA4, TP53, IMPDH2, RPL13, MDM2, CTAG1, RPH3AL, ANXA4, MTDH, TPM3_iso1, ERBB2_C_term, IGF2BP1, MIA, DDX53, HMGN3, CALU, KLK3_iso1, REG3A, CTAG2, ERBB2_N_term, and MAGEA3. Preferred are panels comprising MAGEA4. Other preferred panels comprise MAGEA4 and TP53, or MAGEA4, TP53, and IMPDH2, or MAGEA4, TP53, IMPDH2, and MDM2.

Optionally, the kit can further comprise instructions for suitable operational parameters in the form of a label or a separate insert. For example, the kit may have standard instructions informing a consumer how to wash the probe after a sample of plasma is contacted on the probe.

In either embodiment, the kit may optionally further comprise a standard or control information so that the test sample can be compared with the control information standard to determine if the test amount of a marker detected in a sample is a diagnostic amount consistent with a diagnosis of cancer.

The present invention will now be further described in the following examples with reference to the accompanying figures and sequences, nevertheless, without being limited thereto. For the purposes of the present invention, all references as cited herein are incorporated by reference in their entireties. In the Figures:

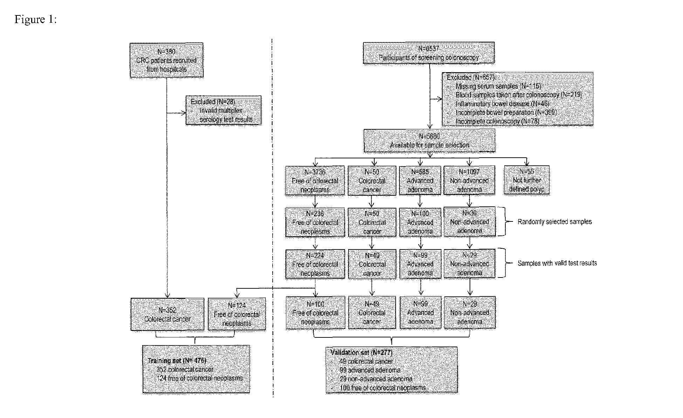

- **Figure 1:**: shows a flow diagram of sample selection procedure of the training set and the validation set.

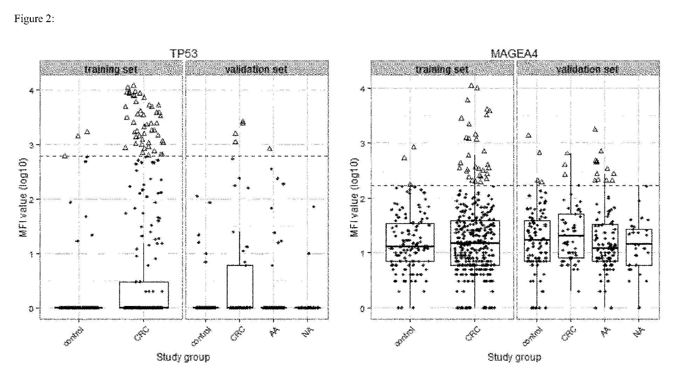

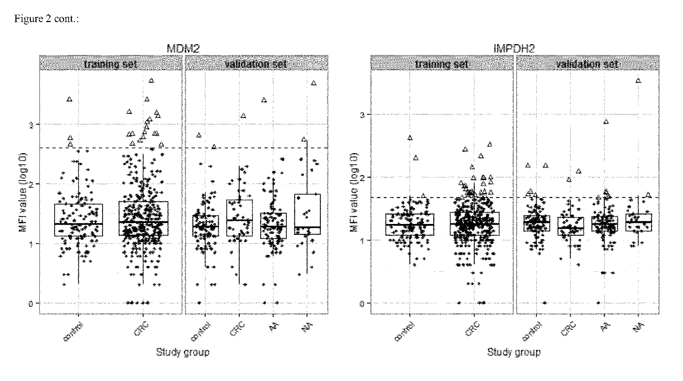

- **Figure 2:**: shows a scatter plot and box plot showing the distribution of median fluorescence intensity (MFI) values (in log10 transformation) of four autoantibody biomarkers (anti-TP53, anti-MAGEA4, anti-MDM2 and anti-IMPDH2) between different study groups. The bottom and top of the box indicate the first (Q1) and third (Q3) quartiles, and the middle line in the box is the median; the upper-limit equals Q3 plus 1.5 times interquartile range (IQR), and the lower-limit equals Q1 minus 1.5 times IQR. The horizontal dashed lines represent the cutoffs of respective biomarkers. Triangles represent samples with MFI values above the respective cutoffs, and solid dots represent samples with MFI values below the respective cutoffs. Abbreviations: AA, advanced adenoma; CRC, colorectal cancer; NA, non-advanced adenoma.

### EXAMPLES

### Materials and Methods

### Study design and study population

This analysis is based on the ongoing BliTz study (Begleitende Evaluierung innovativer Testverfahren zur Darmkrebsfrüherkennung), which has been described in detail elsewhere (13, 14). Briefly, participants of screening colonoscopy are recruited in gastroenterology practices in Southern Germany since November 2005. Patients are recruited at a preparatory visit and invited to donate blood and stool samples for the evaluation of novel CRC screening tests (typically one week) before screening colonoscopy. Colonoscopy records are collected from all participants.

For this analysis, the following exclusion criteria were applied to exclude participants without adequate blood samples, participants not representing a true screening setting, and participants with potentially inadequate colonoscopy results: blood samples taken after screening colonoscopy, history of CRC or inflammatory bowel disease, incomplete colonoscopy or insufficient bowel preparation (latter only for controls). From the remaining participants of the BliTz study recruited in 2005-2013 (N=5680), all 50 newly detected CRC cases, as well as random samples of participants with advanced colorectal adenomas (AA, N=100), non-advanced colorectal adenomas (NA, N=30) and with no colorectal neoplasms (controls, N=236) were included in the measurement. Because in a true screening population like this, CRC patients are expected to be on average slightly older and to include a somewhat larger proportion of men, age and sex were not matched between different disease groups to avoid biased estimates of specificity (15).

Given the limited number of CRC cases even in a screening study as large as the BliTz study, also 380 CRC cases recruited after diagnosis but before treatment at four hospitals in Southern Germany for the discovery phase of this study were recruited.

### Classification of colorectal cancer and advanced adenoma

Based on the colonoscopy reports or hospital records of all participants, invasive CRC was classified into stages Tis (carcinoma in situ) and I-IV according to the UICC (Union for International Cancer Control) TNM (tumor-node-metastasis) stage classification. In addition, advanced adenomas were subclassified into high grade dysplasia (HGD), villous architecture without HGD, and large adenomas (≥10 mm) with neither HGD nor villous architecture. Relevant information was extracted by two research assistants independently who were blind to the blood test results.

### Sample preparation

Blood samples were collected in Sarstedt S-Monovette (Product No. 02.1388) tubes and Vacutainer SST II (Product No. 367953) tubes before bowel preparation for colonoscopy (BliTz study), or prior to large bowel surgery or neoadjuvant therapy (380 CRC cases from the clinical setting). Blood samples were immediately centrifuged at 2000-2500g for 10 minutes and stored at -80°C until analyses. Details on the standard operating procedures have also been described previously (16).

### Multiplex serology

64 candidate TAAs encoded by 59 genes based on previous autoantibody measurements (17) and two recent systematic reviews (8, 10) were selected. Details on the 64 candidate TAAs are provided in Table 6. Autoantibodies to the selected TAAs were measured by multiplex serology, a fluorescent bead-based GST capture immunosorbent assay, as described previously (17, 18). In short, TAAs were bacterially expressed as GST-X-tag fusion proteins (19), loaded and affinity-purified on glutathione-casein-coupled spectrally distinct fluorescence-labeled polystyrene beads (SeroMap, Luminex Corp., Austin, Tx, USA). A mix of differently loaded bead sets provides an antigen suspension array that is presented to sera. A Luminex analyzer (Luminex Corp., Austin, Tx, USA) distinguishes the bead set by its internal bead-color and quantifies the amount of serum-antibody detected by a secondary goat antihuman IgA, IgM, IgG antibody (Dianova, Hamburg, Germany) and the reporter conjugate streptavidin-R-phycoerythrin. The antibody reactivity is given as median fluorescence intensity (MFI) of at least 100 beads per set measured. Final antigen-specific MFI values were generated by subtraction of GST-tag and individual bead background values.

Sera were sent on dry ice to the Division of Molecular Diagnostics of Oncogenic Infections, DKFZ, Heidelberg and analyzed in a 1:1000 dilution by laboratory staff that was blinded for the case-control status.

Antigen-loading of the beads was controlled via detection of the C-terminal tag. Identity of the antigen loaded on the beads was verified by identifying the encoding plasmids via PCR and sequencing. Serum samples were denominated as invalid due to either insufficient amount of serum or too high GST-background values (>300 MFI). Variation between different assay-plates was controlled by pipetting three control sera on each plate as replicates. For overall 19 replicates, coefficients of variation (CV) were calculated for every antigen with a mean reactivity above 30 MFI. The median (range) CVs for the three samples were 16% (10-24%), 14% (11-21%), and 18% (11-25%), respectively.

### Fecal immunochemical test (FIT)

In this study, two types of FTTs, quantitative FIT RIDASCREEN^{®} Hemoglobin (December 2005 to February 2009, R-Biopharm AG, Darmstadt, Germany) and quantitative FIT FOB Gold^{®} (March 2009 until now, Sentinel Diagnostics, Mailand, Italy), were conducted among study participants. Until January 2012, participants were asked to fill containers with raw stools at home and keep them in the freezer until the practice visit. On the day of the visit, stool-filled containers were immediately stored at -20°C in the practice and then shipped on dry ice to a central laboratory for further analysis according to the instructions of the manufacturers. After January 2012, participants were provided buffer-filled stool collection tubes from Sentinel and were asked to mail directly to the central laboratory for further analysis. In order to ensure the comparability of two types of FITs under these three conditions (frozen stool + RIDASCREEN, frozen stool + FOB Gold, and fresh stool in buffer-filled sentinel tube + FOB Gold), cut-offs were defined to yield 95% specificity based on all available BliTz controls under the respective FIT condition.

### Statistical analyses

Applied was a two-step approach with selection of markers and marker combinations in a training set, and validation of the results in an independent validation set. The validation set was defined in such a way that it represents a true screening setting, i.e., only participants from the BliTz study were included. CRC cases recruited in the clinical setting and randomly selected controls from the BliTz study were used for the training set.

Characteristics of the study population were first described and compared between the training set and the validation set. The strategy for selection of autoantibodies and their cutoff levels for predicting presence of colorectal neoplasm were based on the particular character of single autoantibody markers, which typically have rather low sensitivity at very high specificity (8, 10). Cutoff levels yielding 98% specificity in the training set were determined (cutoffs lower than 30 MFI were replaced by a technical minimum cutoff of 30 MFI to reduce the influence of background noise of the multiplex serology measurement). After applying the cutoffs to respective markers, dichotomous variables indicating the predicted outcome (positive or negative) were generated. Sensitivities of all the 64 autoantibody markers for detecting CRC were calculated. Additionally, multi-marker combinations were also explored to enhance the diagnostic performance. Multi-marker combinations were considered positive, if at least one marker in the combination was positive. A systematic search for the best n-marker combination (starting with n=2) was conducted in the following steps: i) calculate the Youden index, equaling the sum of sensitivity for detecting early stage CRC (UICC TNM stage I/II) and specificity minus 1 for all possible marker combinations; ii) determine the specific n-marker combination which gives the highest index and evaluate its diagnostic performance in the training set; iii) continue the selection process with increasing n, the number of markers included in the combination, until the specificity of the best performing marker combination in the training set falls below 90%. The diagnostic performance of the final marker-combination was then evaluated in the validation set. 95% confidence intervals (95% CIs) of sensitivity and specificity were calculated using the Wilson method (20).

The combination of selected best performing multi-marker panel with FIT was also evaluated. The new combination was considered positive if at least one marker or FIT test in the combination was positive. Sensitivities and specificities for the multi-antibody panel, FIT, and the combination of multi-autoantibody panel and FIT were calculated. 95% CIs of sensitivity and specificity were calculated using the Wilson method (20).

All statistical analyses were performed with the statistical software R version 3.1.1(21). All tests were two-sided and p-values of 0.05 or less were considered to be statistically significant.

### Example 1: Serological Autoantibody Diagnostic Markers for Colorectal Cancer

Figure 1 provides the flow diagram showing the study population selection for the training set and the validation set. Overall, 380 clinically identified CRC cases were included in this study. After excluding 28 samples with invalid multiplex serology test results, the remaining 352 CRC patients were included as cases in the training set. The independent validation set samples were exclusively sampled from participants enrolled in the BliTz study in 2005-2013. After excluding participants without adequate blood samples, participants who do not represent a true screening setting, and participants with potentially false negative results at screening colonoscopy, 5680 participants were eligible for the sample selection, from whom 417 samples (all CRC case and random sample of advanced adenomas, non-advanced adenomas and controls free of colorectal neoplasms) were selected for the measurement. After further excluding samples with invalid laboratory results, 49 CRC cases, 99 advanced adenomas, 29 non-advanced adenomas and 224 controls free of colorectal neoplasms were included in the analysis. The average time between blood sample withdrawal and screening colonoscopy among these participants was 6.6 days. The inventors randomly selected 124 controls from this screening setting as control group in the training set. The remaining 100 participants were used as controls in the validation set.

**Table 1. Characteristics of the study population**

| **Group** | **Training set** | | **Validation set** | | | |
|---|---|---|---|---|---|---|
| | **CRC (N, %)** | **Control (N, %)** | **CRC (N, %)** | **Advanced adenoma (N, %)** | **Non-advanced adenoma (N, %)** | **Control (N, %)** |
| **Age (years)** | | | | | | |
| **<60** | 73 (20.8) | 50 (40.3) | 9 (18.4) | 30 (30.3) | 10 (34.5) | 43 (43.0) |
| **60-64** | 49 (14.0) | 30 (24.2) | 14 (28.6) | 27 (27.3) | 7 (24.1) | 22 (22.0) |
| **65-69** | 57 (16.2) | 22 (17.7) | 11 (22.4) | 16 (16.2) | 6 (20.7) | 20 (20.0) |
| **≥70** | 172 (49.0) | 22 | 15 (30.6) | 26 (26.3) | 6 (20.7) | 15 |
| | | (17.7) | | | | (15.0) |
| **Mean ± SD** | 68.3 ± 11.6 | 62.2 ± 7.4 | 66.3 ± 6.7 | 64.5 ± 7.7 | 63.6 ± 6.5 | 62.0 ± 6.1 |
| **Sex** | | | | | | |
| **Male** | 202 (57.4) | 56 (45.2) | 34 (69.4) | 50 (50.5) | 17 (58.6) | 45 (45.0) |
| **Female** | 150 (42.6) | 68 (54.8) | 15 (30.6) | 49 (49.5) | 12 (41.4) | 55 (55.0) |
| **UICC TNM tumor stage** | | | | | | |
| **Tis (0)** | - | - | 4 (8.2) | - | - | - |
| **I** | 96 (27.3) | - | 18 (36.7) | - | - | - |
| **II** | 102 (29.0) | - | 5 (10.2) | - | - | - |
| **III** | 105 (29.8) | - | 19 (38.8) | - | - | - |
| **IV** | 49 (13.9) | - | 3 (6.1) | - | - | - |
| **CRC location** | | | | | | |
| **Colon** | 202 (57.4) | - | 26 (53.1) | - | - | - |
| **Rectum** | 150 (42.6) | - | 22 (44.9) | - | - | - |
| **Unknown** | | - | 1 (2.0) | - | - | - |
| **Advanced adenoma subclass** | | | | | | |
| **HGD** | - | - | - | 12 (12.1) | - | - |
| **Villous** | - | - | - | 55 (55.6) | - | - |
| **Large polyp** | - | - | - | 32 (32.3) | - | - |

| | | | | | | |
|---|---|---|---|---|---|---|
| Abbreviations: CRC, colorectal cancer; HGD, high grade dysplasia; SD, standard deviation; TNM, tumor-node-metastasis; UICC, Union for International Cancer Control. | | | | | | |

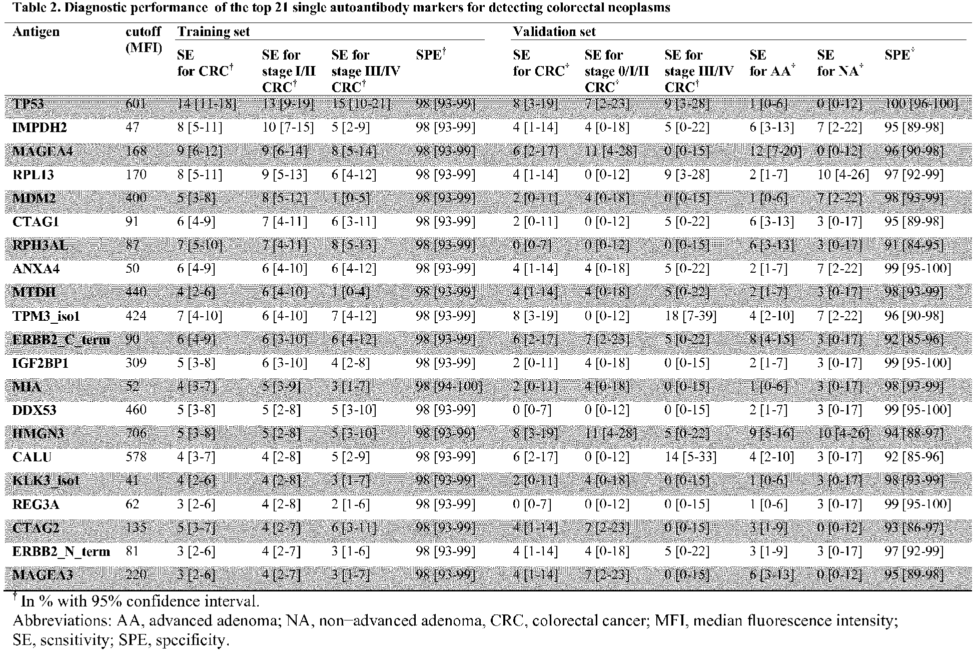

**Table 3. Diagnostic performance of best performing two-, three-, four-, five-, and six-marker combinations derived from training set for detecting colorectal neoplasms**

| **Marker Combination** | **Training set** | | | | **Validation set** | | | | | |
|---|---|---|---|---|---|---|---|---|---|---|
| | **SE for CRC^{†}** | **SE for stage I/II CRC^{†}** | **SE for stage III/IV CRC^{†}** | **SPE ^{†}** | **SE for CRC^{†}** | **SE for stage 0/I/II CRC^{†}** | **SE for stage III/IV CRC^{†}** | **SE for AA^{†}** | **SE for NA^{†}** | **SPE ^{†}** |
| ***Best performing two-marker combination*** | | | | | | | | | | |
| **TP53+IMPDH2** | 22 (18-26) | 23 (17-29) | 20 (15-28) | 95 (90-98) | 10 (4-22) | 11 (4-28) | 9 (3-28) | 7 (3-14) | 7 (2-22) | 95 (89-98) |
| ***Best performing three-marker combination*** | | | | | | | | | | |
| **TP53+IMPDH2+MDM2** | 26 (22-31) | 30 (24-37) | 21 (15-29) | 93 (87-96) | 12 (6-32) | 15 (6-32) | 9 (3-28) | 8 (4-15) | 10 (4-26) | 94 (88-97) |
| ***Best performing four-marker combination*** | | | | | | | | | | |
| **TP53+IMPDH2+MDM2+MAGEA4** | 33 (28-38) | 37 (30-44) | 28 (21-36) | 90 (84-94) | 18 (10-31) | 26 (13-45) | 9 (3-28) | 20 (13-29) | 10 (4-26) | 90 (83-94) |
| ***Best performing five-marker combination*** | | | | | | | | | | |
| **TP53+IMPDH2+MDM2+MAGEA4+ CTAG1** | 38 (33-43) | 43 (36-50) | 32 (25-40) | 89 (82-93) | 20 (11-34) | 26 (13-45) | 14 (5-33) | 24 (17-34) | 14 (5-31) | 87 (79-92) |
| ***Best performing six-marker combination*** | | | | | | | | | | |
| **TP53+IMPDH2+MDM2+MAGEA4+ CTAG1+MTDH** | 40 (34-45) | 46 (39-53) | 32 (25-40) | 88 (81-93) | 24 (15-38) | 30 (16-48) | 18 (7-39) | 25 (18-35) | 17 (8-35) | 85 (77-91) |

| | | | | | | | | | | |
|---|---|---|---|---|---|---|---|---|---|---|
| ^{†} In % with 95% confidence interval. Abbreviations: AA, advanced adenoma; CI, confidence interval; CRC, colorectal cancer; NA, non-advanced adenoma; SE, sensitivity; SPE, specificity. | | | | | | | | | | |

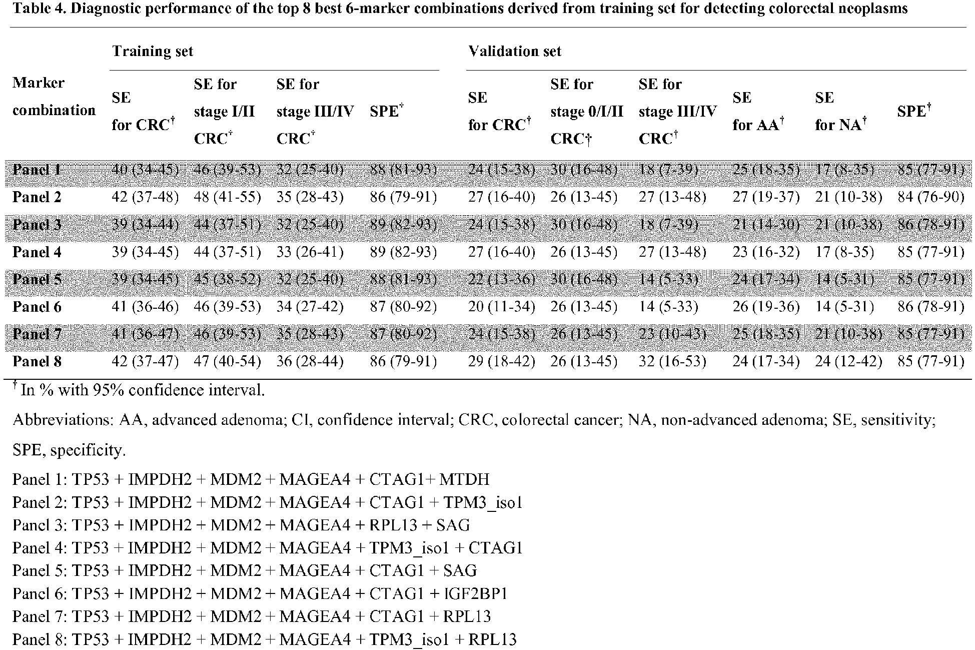

The diagnostic performance of the 21 best performing autoantibodies identified in the training set is presented in Table 2. Using cutoffs yielding 98% specificity in the training set generally resulted in rather low levels of sensitivity in both the training set and the validation set. The majority of markers were found to have lower sensitivities for CRC in the validation set compared to the training set. Overall, there were 21 markers showing sensitivities for early stage CRC ≥4% at 98% specificity in the training set. However 7 of them did not detect any early stage CRC in the validation set (see Table 2). The inventors further examined the joint seropositivity of the top 21 markers among cases of the training set. There was only very limited co-occurrence beyond chance of the single autoantibody markers. Although the majority of Kappa coefficients were positive (145 out of 210, 69%), 189 out of 210 Kappa coefficients (90%) for pairwise joint occurrence were close to zero with values between -0.10 and +0.20.

### Example 2: Anti-Mage A4 as Diagnostic Marker for Early Stage CRC and Advanced Adenomas

Overall, there were only 9 autoantibody markers whose sensitivities for detecting early stage CRC were higher than 5%, ranging from 7% to 11% in the validation set. Anti-MAGEA4 was found to have best diagnostic potential for detecting early stage CRC and advanced adenomas, with sensitivities of 11% (95% CI, 4-28%) and 12% (95% CI, 7-20%), respectively, at a specificity of 96%. Anti-TP53 detected 8% of CRC (7% at early stage), but only 1% of advanced adenomas at 100% specificity.

### Example 3: Diagnostic Autoantibody Marker Panels

A systematic search for the best n-marker combination was conducted among all the 64 autoantibody markers with the purpose to enhance the diagnostic performance. Results for the best performing 2-, 3-, 4-, 5- and 6-marker combinations are shown in Table 3. Including six or more markers in the combination resulted in specificities much lower than 90% for the best performing marker combinations even in the training set, the analysis was therefore limited to combinations of no more than 6 markers. As expected, sensitivities increased and specificities decreased with increasing numbers of markers included the multi-marker combination. For example, sensitivity for any CRC increased from 22% for the best performing 2-marker panel to 40% for the best performing 6-marker panel in the training set, whereas specificity decreased from 95% to 88%. Interestingly, sensitivity was consistently higher for early stage than for advanced stage CRC. As expected, sensitivities were slightly lower in the validation set, but a sensitivity of 30% (95% CI, 16-48%) for early stage CRC along with a specificity of 85% (95% CI, 77-91%) was still observed for the best performing 6-marker combination. Additionally, it also detected 25% (95% CI, 18-35%) of advanced adenomas, the most important precursors of CRC.

Further evaluated were additional seven 6-marker combinations, whose Youden indices were only slightly lower than the best-performing 6-marker combination in the training set. Details of the diagnostic performance of these combinations are shown in Table 4. Overall, the sensitivities of these 6-marker combinations for detecting CRC ranged from 20% to 29% at specificities ranging from 84% to 86%. The sensitivities for detecting early stage CRC were higher than for detecting advanced stage CRC, and ranged from 26% to 30%. Notably, these panels also exhibited good diagnostic potential for detecting advanced adenomas, with sensitivities ranging from 21% to 27%. Interestingly, four autoantibody markers, anti-TP53, anti-IMPDH2, anti-MDM2 and anti-MAGEA4, were consistently included in all of these seven 6-marker combinations. These are also the makers that were included in the best 4-marker combination which yielded almost the same sensitivity for early stage and advanced adenomas (26% and 20%, respectively), albeit at higher specificity (90%). A graphic presentation of the MFI value distributions of these four markers is shown in Figure 2.

### Example 4: Combination of the Four-autoantibody Diagnostic Panel and FIT

Table 5 presents a comparison of diagnostic performance of detecting colorectal neoplasms among the 4-autoantibody panel (anti-TP53, anti-IMPDH2, anti-MDM2 and anti-MAGEA4), FIT, and the combination of 4-autoantibody panel and FIT. Surprisingly, a substantially higher sensitivity for detecting CRC was observed for the FIT compared to the 4-marker panel. Additionally, the FIT exhibited higher sensitivity for detecting CRC recruited in the screening setting compared to CRC recruited in the clinical setting, while an opposite trend was observed for the 4-autoantibody panel. For the 4-autoantibody panel, the sensitivity for detecting advanced adenomas was 19.4% (95% CI, 12.8-28.3%), which was only slightly lower than the sensitivity of FIT (29.6%, 95% CI, 21.5-39.3%).

Combining FIT with the 4-autoantibody panel yielded an improvement in terms of sensitivities for detecting colorectal neoplasms (Table 5). The greatest improvement was observed for detecting advanced adenomas, from 29.6% of FIT solely to 42.9% of the new combination. Sensitivities of this combination for detecting different subclasses of advanced adenomas did not vary greatly, with sensitivities for detecting HGD, villous architecture without HGD, and large polyps (≥10 mm) of 40.0% (6/12), 35.2% (19/54), and 53.1% (17/32), respectively.

**Table 5. Diagnostic performance of different tests in detecting colorectal neoplasms**

| **Test** | **Sensitivity** | | | | **Specificity** |
|---|---|---|---|---|---|
| | **(95% CI, %)** | | | | **(95% CI, %)** |
| | **CRC (Clinical)** | **CRC (Screening)** | **Advanced adenoma** | **Non**-**advan ced adenoma** | **Control** |
| **4-marker panel** | 32.4 (24.1-41.9) | 17.8 (9.3-31.3) | 19.4 (12.8-28.3) | 10.3 (3.6-26.4) | 90.0 (82.6-94.5) |
| **FIT** | 73.5 (64.2-81.1) | 86.7 (73.8-93.7) | 29.6 (21.5-39.3) | 13.8 (5.5-30.1) | 97.0 (91.5-99.0) |
| **4-marker panel + FIT** | 83.3 (74.9-89.3) | 88.9 (76.5-95.2) | 42.9 (33.5-52.7) | 24.1 (12.2-42.1) | 87.0 (79.0-92.2) |

| | | | | | |
|---|---|---|---|---|---|
| 4-Marker panel: anti-TP53, anti-IMPDH2, anti-MDM2 and anti-MAGEA4 FIT: Fecal Immunochemical Test | | | | | |

**Table 6. Gene names and protein names of all 64 measured antigens**

| **Synonyms** | **Protein full names** | **Protein length (amino acid sequence)** |
|---|---|---|
| **ACRBP** | Acrosin-binding protein | full length |
| **AIMP1** | Aminoacyl tRNA synthase complex-interacting multifunctional protein 1 | full length |
| **ANXA4** | Annexin A4 | full length |
| **BIRC5** | Baculoviral IAP repeat-containing protein 5 | full length |
| **CALU** | Calumenin | full length |
| **CAMEL** | CTL-recognized antigen on melanoma | full length |
| **CCNB1** | G2/mitotic-specific cyclin-B1 | full length |
| **CCND1** | G1/S-specific cyclin-D1 | full length |
| **CDKN2A** | Cyclin-dependent kinase inhibitor 2A | full length |
| **CEACAM5** | Carcinoembryonic antigen-related cell adhesion molecule 5 | full length |
| **CT47A** | Cancer/testis antigen 47A | full length |
| **CTAG1** | Cancer/testis antigen 1 | full length |
| **CTAG2** | Cancer/testis antigen 2 | full length |
| **DDX53** | Probable ATP-dependent RNA helicase DDX53 | full length |
| **EGFR_C_term** | Epidermal growth factor receptor | 669-1210 |
| **ERBB2_N_term** | Receptor tyrosine-protein kinase erbB-2 | 23-652 |
| **ERBB2_C_term** | Receptor tyrosine-protein kinase erbB-2 | 676-1255 |
| **FOLH1_iso1** | Glutamate carboxypeptidase 2 (isoform 1) | full length |
| **FOLH1_iso7** | Glutamate carboxypeptidase 2 (isoform 7) | full length |
| **GAGE7** | G antigen 7 | full length |
| **GRINA** | Protein lifeguard 1 | full length |
| **GRINA_N-term** | Protein lifeguard 1 | 1-164 |
| **HIST1H2B** | Histone H2B type 1-C/E/F/G/I | full length |
| **HMGN3** | High mobility group nucleosome-binding domain-containing protein 3 | full length |
| **HSPA2** | Heat shock-related 70 kDa protein 2 | full length |
| **HSPA5** | 78 kDa glucose-regulated protein | 19-end |
| **IGF2BP1** | Insulin-like growth factor 2 mRNA-binding protein 1 | full length |
| **IGF2BP3** | Insulin-like growth factor 2 mRNA-binding protein 3 | full length |
| **IMPDH2** | Inosine-5'-monophosphate dehydrogenase 2 | full length |
| **KLK3_iso1** | Prostate-specific antigen (isoform 1) | full length |
| **KLK3_iso2** | Prostate-specific antigen (isoform 2) | full length |
| **KRAS** | GTPase Kras | 1-37 |
| **MAGEA1** | Melanoma-associated antigen 1 | full length |
| **MAGEA3** | Melanoma-associated antigen 3 | full length |
| **MAGEA4** | Melanoma-associated antigen 4 | full length |
| **MAPKAPK3** | MAP kinase-activated protein kinase 3 | full length |
| **MDM2** | E3 ubiquitin-protein ligase Mdm2 | full length |
| **MLANA** | Melanoma antigen recognized by T-cells 1 | full length |
| **MPHOSPH6** | M-phase phosphoprotein 6 | full length |
| **MIA** | Melanoma-derived growth regulatory protein | full length |
| **MTDH** | Protein LYRIC | 271-451 |
| **MUC1_iso8** | Mucin-1 (isoform 8) | full length |
| **MYC** | Myc proto-oncogene protein | full length |
| **PMEL** | Melanocyte protein PMEL | full length |
| **PSCA** | Prostate stem cell antigen | full length |
| **REG3A** | Regenerating islet-derived protein 3-alpha | 27-end |
| **RPH3AL** | Rab effector Noc2 | full length |
| **RPL13** | 60S ribosomal protein L13 | full length |
| **SNAP25** | Synaptosomal-associated protein 25 | full length |
| **SAG** | S-arrestin | full length |
| **SDCCAG8** | Serologically defined colon cancer antigen 8 | 225-537 |
| **SEC61B** | Protein transport protein Sec61 subunit beta | full length |
| **SPAG9** | C-Jun-amino-terminal kinase-interacting protein 4 | full length |
| **SPANXA** | Sperm protein associated with the nucleus on the X chromosome A | full length |
| **SSX2** | Protein SSX2 | full length |
| **SSX4** | Protein SSX4 | full length |
| **TP53** | Cellular tumor antigen p53 | full length |
| **TPM3_iso1** | Tropomyosin alpha-3 chain (isoform 1) | full length |
| **TPM3_iso3** | Tropomyosin alpha-3 chain (isoform 3) | full length |
| **TRP-2** | Tyrosinase-related protein-2 | full length |
| **UBE2D1** | Ubiquitin-conjugating enzyme E2 D1 | full length |
| **UBQLN1** | Ubiquilin-1 | full length |
| **UCHL3** | Ubiquitin carboxyl-terminal hydrolase isozyme L3 | full length |
| **VIL1** | Villin-1 | full length |

### Discussion

In this study, autoantibody markers and their combinations for early detection of CRC and its precursors in a large set of samples were identified, which were then independently validated in prospectively collected samples from a true screening population. Overall, most of the tested autoantibodies showed relatively low sensitivity at cutoff levels yielding high specificity. Among all single markers, anti-MAGEA4 showed the highest sensitivity for detecting early stage CRC (11%, 95% CI, 4-28%) and advanced adenomas (12%, 95% CI, 7-20%) at a specificity of 96% (95% CI, 90-98%). The inventors also explored the potential of multi-marker combinations to enhance diagnostic performance. Sensitivity increased and specificity decreased with increasing numbers of markers included in multi-marker combinations. All marker-combinations had higher sensitivities for early stage than for advanced stage CRC even in the validation set. Four markers, including anti-TP53, anti-MAGEA4, anti-MDM2 and anti-IMPDH2, were consistently included in best performing 4-, 5-, and 6-marker combinations. A combination of these four markers could detect 26% of early stage CRC (95% CI, 13-45%) and 20% of advanced adenomas (95% CI, 13-29%) at a specificity of 90% (95% CI, 83-94%). The potential combination of these four autoantibody markers with FIT was also evaluated, with improved sensitivities for detecting advanced adenomas (42.9%, 95% CI: 33.5-52.7%) and non-advanced adenomas (24.1%, 95% CI: 12.2-42.1 %), which were higher than respective sensitivities of FIT.

In the present analyses, a two-step approach was taken including selecting biomarkers in a training set and subsequently validating findings in an independent validation set. Of note, the validation was performed in a true screening setting, i.e., in the ideal target population for CRC screening, with colonoscopic verification of presence or absence of colorectal neoplasms (including adenomas) among all participants. By adopting such a study design and analysis strategy, the pitfalls often encountered in retrospective study designs could be avoided. In addition, a multiplex Luminex-based serological assay was applied to measure all 64 autoantibody markers. Previous research results indicated that multiplex serology exhibited increased detection of weak antibody responses compared to ELISA, thus possibly providing more accurate results regarding serum autoantibody responses against TAAs.

Among tested single autoantibody markers, anti-MAGEA4 was found to have good diagnostic potential for detecting early stage CRC (sensitivity=11%; 95% CI, 4-28%) and advanced adenomas (sensitivity=12%; 95% CI, 7-20%) in this analysis. MAGEA4 is a member of cancer/testis (CT) antigens, which is expressed in malignant cells and in immune-privileged germ-line cells. It has a putative role of mediating anti-apoptotic functions by interacting with TP53. Over-expression of MAGEA4 was previously reported in other malignancies, such as lung cancer and ovarian cancer. This is the first study to indicate that humoral responses to MAGEA4 could be observed in the early stage of CRC and its precursors. In particular it has potential to be included in a multi-marker panel.

As genetic or epigenetic alterations leading to aberrantly expression of antigens, such as TP53 (34) and CTAG1 (also known as NY-ESO-1), may occur in various types of cancer, humoral responses to autoantigens evaluated in the present study may also be observed in patients with other malignancies rather than in CRC only. To what extent these markers could detect other malignancies should be evaluated in future studies.

Notably, sensitivities for detecting advanced adenomas (≥20%) of the marker combinations were similar to sensitivities reported for FITs, and clearly superior to sensitivities reported for gFOBTs and any of other suggested blood tests that were validated in screening settings. For instance, a recent study by Church and colleagues tested methylated Septin9 in 7941 asymptomatic individuals attending screening colonoscopy, and reported a sensitivity for detecting advanced adenoma of 4.2% (6/130) and 15.7% (24/184) for women and men, respectively, at a specificity of 91.5%.

In the present study, the inventors deliberately selected antibody markers against TAAs based on their ability to detect early stage CRC rather than any stage CRC. This most likely explains the apparently unusual finding of better diagnostic performance for early stage CRC than for late stage CRC even in the validation set, whereas other biomarker studies typically showed opposite patterns. Without being bound to any theory, another possible explanation might be immune destruction, an emerging hallmark of cancer, which occurs in advanced stage of CRC inducing the loss of antibody production. In a screening setting, the vast majority of prevalent preclinical and previously undetected CRC are at an early stage. Early detection and removal of these cancers, along with early detection and removal of the precursors, advanced adenomas, is likely to bear the largest potential for prevention of CRC deaths.

## Claims

1. A *non-invasive* method for, or for aiding, a diagnosis, prognosis, stratification and/or monitoring of a therapy, of a cancer disease in a subject, comprising the steps of:
(a) Providing a biological sample from the subject,
(b) Determining in the sample the level of at least one autoantibody selected from anti-MAGEA4, anti-TP53, anti-IMPDH2, anti-RPL13, anti-MDM2, anti-CTAG1, anti-RPH3AL, anti-ANXA4, anti-MTDH, anti-TPM3_iso1, anti-ERBB2_C_term, anti-IGF2BP1, anti-MIA, anti-DDX53, anti-HMGN3, anti-CALU, anti-KLK3_iso1, anti-REG3A, anti-CTAG2, anti-ERBB2_N_term, or anti-MAGEA3,
wherein a differential level of the at least one autoantibody in the biological sample from the subject as determined in step (b) compared to a healthy control sample or reference value is indicative for the presence of the cancer disease in the subject.

2. The method according to claim 1, wherein step (b) comprises determining in the sample the level of autoantibodies anti-TP53 and anti-IMPDH2.

3. The method according to claim 1 or 2, wherein step (b) comprises determining in the sample the level of autoantibodies anti-TP53, anti-IMPDH2 and anti-MDM2; or anti-MAGEA4, anti-TP53, anti-IMPDH2, and anti-MDM2; or anti-MAGEA4, anti-TP53, anti-IMPDH2, anti-MDM2, and anti-CTAG1.

4. The non-invasive method according to any of claims 1 to 3, wherein step (b) comprises determining in the sample the level of the autoantibodies anti-MAGEA4, anti-TP53, anti-IMPDH2, and anti-MDM2, anti-CTAG1 and anti-MTDH.

5. The method according to claim 1, wherein step (b) comprises determining in the sample the level of anti-MAGEA4 autoantibodies, and at least one further autoantibody selected from anti-TP53, anti-IMPDH2, anti-RPL13, anti-MDM2, anti-CTAG1, anti-RPH3AL, anti-ANXA4, anti-MTDH, anti-TPM3_iso1, anti-ERBB2_C_term, anti-IGF2BP1, anti-MIA, anti-DDX53, anti-HMGN3, anti-CALU, anti-KLK3_iso1, anti-REG3A, anti-CTAG2, anti-ERBB2_N_term, or anti-MAGEA3.

6. The method according to claim any of claims 1 to 5, wherein step (b) comprises determining in the sample the level of at least one further autoantibody selected from anti-MAGEA4, anti-TP53, anti-IMPDH2, anti-RPL13, anti-MDM2, anti-CTAG1, anti-RPH3AL, anti-ANXA4, anti-MTDH, anti-TPM3_iso1, anti-ERBB2_C_term, anti-IGF2BP1, anti-MIA, anti-DDX53, anti-HMGN3, anti-CALU, anti-KLK3_iso1, anti-REG3A, anti-CTAG2, anti-ERBB2_N_term, or anti-MAGEA3.

7. The method according to any of claims 1 to 6, wherein the biological sample is a tissue sample or body liquid sample, preferably a blood sample, most preferably a serum sample.

8. The method according to any of claims 1 to 7, wherein the method is a screening method for establishing a first diagnosis of cancer in the subject.

9. The method according to any of claims 1 to 8 wherein the cancer is colorectal cancer.

10. The method according to claim 9, wherein the method further comprises the detection of the presence of blood in a stool sample of the subject, preferably by using a fecal immunochemical test (FIT).

11. The method according to claim 9 or 10, wherein the colorectal cancer is early stage 0/I/II colorectal cancer or late stage III/IV colorectal cancer.

12. The method according to any one of claims 1 to 11, wherein a differential level of an autoantibody is a higher level compared to the control or reference level.

13. The method according to any one of claims 1 to 12, wherein the autoantibody is detected using a recombinantly expressed antigen corresponding to, or detectable by, the corresponding autoantibody, preferably wherein the autoantibody is detected via a fluorescent based multiplex immunosorbent assay.

14. A diagnostic kit for performing a method according to any one of claims 1 to 13.

15. Use of a protein, or a fragment thereof, detectable by, or being bound by, an autoantibody selected from anti-MAGEA4, anti-TP53, anti-IMPDH2, anti-RPL13, anti-MDM2, anti-CTAG1, anti-RPH3AL, anti-ANXA4, anti-MTDH, anti-TPM3_iso1, anti-ERBB2_C_term, anti-IGF2BP1, anti-MIA, anti-DDX53, anti-HMGN3, anti-CALU, anti-KLK3_iso1, anti-REG3A, anti-CTAG2, anti-ERBB2_N_term, or anti-MAGEA3, in the performance of a method according to any of claims 1 to 13.
